# EUROPÄISCHE PATENTSCHRIFT

(11) **EP 0 450 608 B1**
(45) Veröffentlichungstag und Bekanntmachung des Hinweises auf die Patenterteilung: **13.07.1994**
(21) Anmeldenummer: 91105297.5
(22) Anmeldetag: 04.04.1991
(51) Int. Cl.: A61B 17/28, A61B 17/32

(54) **Medizinische Zange**
Medical forceps
Pince médicale

(30) Priorität: 04.04.1990 DE 4010775
(43) Veröffentlichungstag der Anmeldung: 09.10.1991
(73) Patentinhaber: Richard Wolf GmbH, 75438 Knittlingen (DE)
(72) Erfinder: Falk, Ernst, W-7137 Sternenfels-Diefenbach (DE); Dingler, Andreas, W-7534 Birkenfeld (DE); Hiltebrandt, Siegfried, W-7134 Knittlingen (DE)
(74) Vertreter: Vollmann, Heiko, Dipl.-Ing.

(56) Entgegenhaltungen:
- EP-A- 0 351 165
- DE-A- 2 106 263
- DE-A- 2 736 707
- DE-A- 3 601 166
- US-A- 3 176 689

## Beschreibung

Die Erfindung betrifft eine medizinische Zange mit einem festen Maulteil, dem ein festes Griffteil zugeordnet ist, und einem schwenkbaren Maulteil, das mittels eines schwenkbaren Griffteils betätigbar ist, wobei das schwenkbare Griffteil in seinem Schaftbereich geteilt ausgebildet ist und die beiden Schaftteile gelenkig miteinander verbunden sind und wobei die Schließkraft des schwenkbaren Griffteiles zur Verringerung der Schließbewegung des schwenkbaren Maulteiles mittels eines zwischen den zwei Schaftteilen des schwenkbaren Griffteils angeordneten Federgliedes begrenzt wird.

Bekannte Zangen dieser Art sind aus der DE-A 36 01 166 zu entnehmen, wo ein festes und ein schwenkbewegliches Maulteil einem festen bzw. einem schwenkbaren Griffteil Zugeordnet ist, wobei zwecks Begrenzung der an den Maulteilen erzeugbaren Druckkraft der letztgenannte Griffteil in zwei gelenkig miteinander verbundene Teilstücke aufgetrennt ist, die gegeneinander mittels eines Federgliedes abgestützt werden.

Bei Überschreiten eines bestimmten Druckes wird bei dieser Ausführung zwar das untere Teilstück gegenüber dem anderen dann nicht mehr verschwenkbaren Teilstück ausgelenkt, beim weiteren Aufeinanderzubewegen der Griffteile nimmt jedoch die alte Kraft der Maulteile entsprechend der Kennlinie des Federgliedes weiter zu. Die sich so erhöhende Kraft kann letztlich zur Perforation oder gar zum Durchtrennen des zwischen den Maulteilen befindlichen Gewebes führen.

Der Erfindung liegt daher die Aufgabe zugrunde, die Zange nach dem vorstehend beschriebenen Stand der Technik dahingehend zu verbessern, daß auf die beiden Maulteile eine nahezu gleichbleibende Kraft ausgeübt werden kann, unabhängig davon, wie weit die beiden Griffteile aufeinander zu bewegt werden.

Diese Aufgabe wird erfindungsgemäß durch den kennzeichnenden Teil des Anspruchs 1 gelöst.

Die erfindungsgemäß ausgebildete Zange ist nachstehend anhand eines in der Zeichnung dargestellten Ausführungsbeispiels näher erläutert. Es zeigen
- Figur 1: eine Gesamt-Seitenansicht der Zange,
- Figur 2: den erfindungsgemäß ausgestalteten Teil der Zange nach Figur 1 in vergrößertem Maßstab dargestellt.

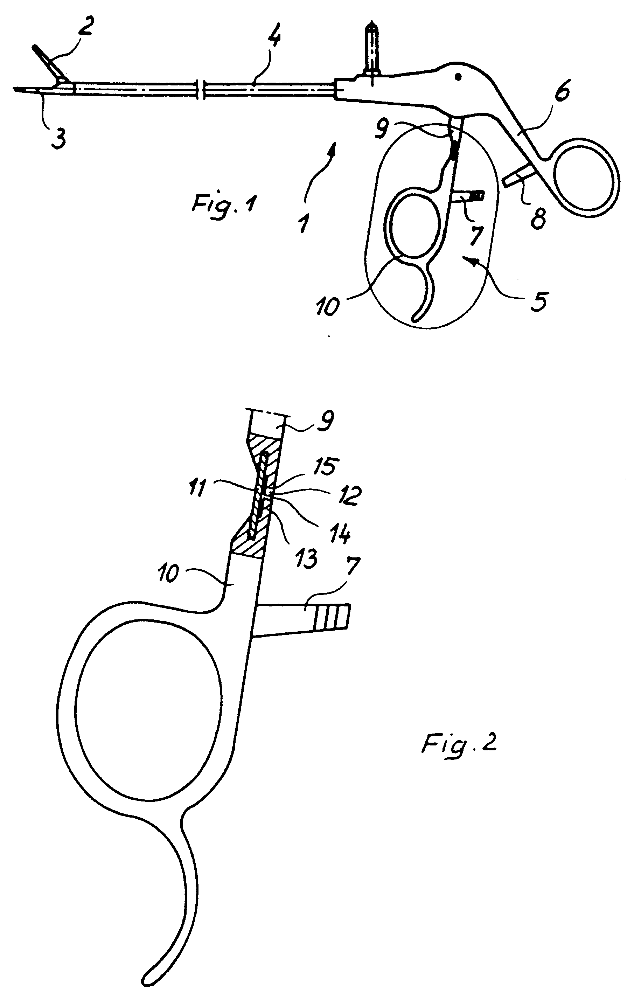

Die Figur 1 zeigt eine Zange 1 mit einem starren Maulteil 3 und einem schwenkbeweglichen Maulteil 2, das über eine in dem Zangenschaft 4 geführte, nicht dargestellte Zug- und Druckstange mittels eines Griffteils 5 unter Gegenhaltung gegen ein starres Griffteil 6 betätigbar ist. Um die beiden Maulteile 2und 3 in der das Gewebe festlegenden Lage fixieren zu können, weisen die beiden Griffteile 5 und 6 Rastelemente 7 und 8 auf, die über eine profiliert ausgebildete Oberfläche miteinander in lösbarem Eingriff bringbar sind.

Das Griffteil 5 besteht aus einem inneren Teil 9 und einem äußeren Teil 10, die über eine Flachfeder 11 unlösbar miteinander verbunden sind. Die Flachfeder 11 wird durch neben dieser angeordnete Stütznasen 12 und 13 bis auf einen geringen Spalt 14 überbrückt, wobei zwischen den Stütznasen 12 und 13 und der Flachfeder 11 ein geringfügiger Luftspalt 15 verbleibt.

Wird mit den beiden Maulteilen 2 und 3 Gewebe gegriffen, welches eine bestimmte Dicke nicht überschreitet, so wird der Spalt 14 zwischen den beiden Stütznasen 12 und 13 der Teile 9 bzw. 10 zwar verringert, und das auch dann, wenn sich die beiden Rastelemente 7 und 8 in Eingriff befinden, ein Aneinanderstoßen der den Spalt 14 begrenzenden Flächen der Stütznasen 12 und 13 erfolgt jedoch nicht. Bei nichtbestimmungsgemäßer Verwendung der Zange, d.h. wenn zuviel Gewebe oder beispielsweise Knorpel mit den beiden Maulteilen 2 und 3 gegriffen wird, ist die zum Schließen derselben erforderliche Kraft so groß, daß die beiden Stütznasen 12 und 13 im Bereich des Spaltes 14 aneinanderstoßen und dadurch einen eine bleibende Verformung des Federelementes verhindernden Sicherheitsanschlag bilden und die auf den äußeren Teil 9 unmittelbar auf das schwenkbewegliche Maulteil 2 übertragen wird. In einem derartigen Fall kann dann natürlich nicht vermieden werden, daß die Nachteile des bekannten Standes der Technik in Erscheinung treten.

## Patentansprüche

1. Medizinische Zange mit einem festen Maulteil (3), dem ein feststehendes Griffteil (6) zugeordnet ist, und einem schwenkbaren Maulteil (2), das mittels eines schwenkbaren Griffteiles (5) betätigbar ist, wobei das schwenkbare Griffteil (5) in seinem Schaftbereich geteilt ausgebildet ist und die beiden Schaftteile (9, 10) gelenkig miteinander verbunden sind und wobei die Schließkraft des schwenkbaren Griffteiles (5) zur Verringerung der Schließbewegung des schwenkbaren Maulteiles (2) mittels eines zwischen den zwei Schaftteilen des schwenkbaren Griffteils (5) angeordneten Federgliedes begrenzt wird, dadurch gekennzeichnet, daß das Federglied ein Biegefederelement (11) ist und sich die beiden zur gelenkigen Verbindung gerichteten Enden der beiden Schaftteile (9, 10) des schenkbaren Griffteiles (5) mit Abstand (14) fluchtend gegenüberliegen und durch das Biegefederelement (11) unlösbar miteinander verbunden sind, dessen Biegeebene mit der Schwenkebene des schwenkbaren Griffteiles (5) zusammenfällt, und daß das äußere Schaftteil (10) des Griffteiles (5) und das feststehende Griffteil (6) je ein Rastelement (7 bzw. 8) zur gegenseitigen Verrastung der Griffteiles (5, 6) aufweisen.

2. Medizinische Zange nach Anspruch 1, dadurch gekennzeichnet, daß die sich gegenüberliegenden Enden der beiden Schaftteile (9, 10) des schwenkbaren Griffteiles (5) auf der festehenden Griffteil (6) zugekehrten Seite je eine Stütznase (12, 13) zur Begrenzung der Auslenkung des Biegefederelementes (11) aufweisen.

## Claims

1. Medical forceps with a fixed jaw portion (3) with which is associated a stationary handle portion (6) and a pivotable jaw portion (2) which can be operated by means of a pivotable handle portion (5) wherein the pivotable handle portion (5) divided in its shaft region and the two shaft portions (9, 10) are hinged together and wherein the closing force of the pivotable handle portion (5) to decrease the closing movement of the pivotable jaw portion (2) is limited by means of a spring member arranged between the two shaft portions of the pivotable handle portion (5), characterised in that the spring member is a leaf spring element (11) and the two ends of the two shaft portions (9, 10) of the pivotable handle portion (5) which are directed towards the hinge joint are located opposite and in alignment at a distance (14) and connected to each other non-releasably by a leaf spring element (11) of which the bending plane coincides with the pivot plane of the pivotable handle portion (5), and in that the outer shaft portion (10) of the handle portion (5) and the stationary handle portion (6) each comprise a latching element (7 or 8) for mutual latching of the handle portions (5, 6).

2. Medical forceps according to claim 1, characterised in that the opposite ends of the two shaft portions (9, 10) of the pivotable handle portion (5) on the side facing towards the stationary handle portion (6) each comprises a supporting lug (12, 13) for limiting the deflection of the leaf spring element (11).

## Revendications

1. Pince médicale, avec une partie de mâchoire fixe, à laquelle est associée une partie de prise fixe, et une partie de mâchoire pivotante, pouvant être actionnée au moyen d'une partie de prise pivotante, la partie de prise pivotante étant réalisée en étant divisée dans sa zone de queue et les deux parties de queue étant reliées ensemble de façon articulée, et la force de fermeture de la partie de prise pivotante étant limitée, en vue de limiter le déplacement d fermeture de la partie de mâchoire pivotante, au moyen d'un organe élastique disposé entre les deux parties de queue de la partie de prise pivotante, caractérisée en ce que l'organe élastique est un élément à ressort de flexion (11) et les deux extrémités, orientées en vue d'assurer une liaison articulée, des deux parties de queue (9, 10) de la partie de prise (5) pivotante étant placées en regard et alignées, espacées d'une distance (14), et reliées ensemble de façon inséparable par un élément de ressort de flexion (11), dont le plan de flexion coïncide avec le plan de pivotement de la partie de prise (5) pivotante, et en ce que la partie de queue extérieure (10) de la partie de prise (5) et la partie de prise (6) fixe présentent chacune un élément d'encliquetage (7, respectivement 8) en vue d'assurer un encliquetage mutuel des parties de prise (5, 6).

2. Pince médicale selon la revendication 1, caractérisée en ce que les extrémités, placées en regard, des deux parties de queue (9, 10) de la partie de prise (5) pivotante présentent chacune, du côté tourné vers la partie de prise (6) fixe, un ergot d'appui (12, 13) destiné à la limitation de la déviation de l'élément élastique de flexion (11).
